# EUROPEAN PATENT SPECIFICATION

(11) **EP 3 474 874 B1**
(45) Date of publication and mention of the grant of the patent: **13.12.2023**
(21) Application number: 17761140.7
(22) Date of filing: 22.06.2017
(51) Int. Cl.: A61K 36/752, A61K 36/8962, A61K 36/9066, A61K 36/185, A61K 36/28, A61K 36/481, A61K 36/534, A61K 36/535, A61K 36/61, A61K 36/71, A61K 36/74, A61P 37/08

(54) **PERILLA EXTRACT COMPOSITION**
PERILLA-EXTRAKT-ZUSAMMENSETZUNG
COMPOSITION D'EXTRAIT DE PÉRILLA

(30) Priority: 28.06.2016 MY PI2016702386
(43) Date of publication of application: 01.05.2019
(73) Proprietor: WindStar Medical GmbH, 61273 Wehrheim (DE)
(72) Inventor: CHEW, Audrey Li Chin, 50480 Kuala Lumpur (MY); CHEN, Qingwen, 50480 Kuala Lumpur (MY); TAN, Bee Kwan, 50480 Kuala Lumpur (MY); SCHEHLMANN, Volker, 50480 Kuala Lumpur (MY); MILLER, Albrecht Jörg Matthias, 50480 Kuala Lumpur (MY)
(74) Representative: Haseltine Lake Kempner LLP
(86) International application number: PCT/MY2017/000024
(87) International publication number: WO 2018/004331

(56) References cited:
- WO-A1-2010/002406
- JP-A- H04 290 822
- JP-A- 2000 316 473
- US-A1- 2009 155 392
- US-B2- 7 384 656
- "Edible oil manufacture useful as nutrient and therapeutic agent for treating cancer, involves extracting seeds of sesame, perilla, eveni primrose, borage and linseed and refining extracted oil", DERWENT, 21 November 2000 (2000-11-21), XP002269468,
- KO JUNG-A ET AL: "Effects of Perilla frutescens Extract on Anti-allergic Reactions in a Mouse Model", KOREAN JOURNAL OF FOOD SCIENCE AND TECHNOLOGY, SEOUL, KR, vol. 42, no. 4, 31 August 2010 (2010-08-31), pages 488-493, XP053024310, ISSN: 0367-6293
- HIROSHI SHIMODA ET AL: "Anti Type I Allergic Property of Japanese Butterbur Extract and Its Mast Cell Degranulation Inhibitory Ingredients", JOURNAL OF AGRICULTURAL AND FOOD CHEMISTRY, vol. 54, no. 8, 1 April 2006 (2006-04-01), pages 2915-2920, XP055112843, ISSN: 0021-8561, DOI: 10.1021/jf052994o
- SZELENYI I ET AL: "HERBAL REMEDIES FOR ASTHMA TREATMENT: BETWEEN MYTH AND REALITY", DRUGS OF TODAY / MEDICAMENTOS DE ACTUALI, J.R. PROUS SS.A. INTERNATIONAL PUBLISHERS, ES, vol. 38, no. 4, 1 April 2002 (2002-04-01), pages 265-303, XP008007606, ISSN: 0025-7656, DOI: 10.1358/DOT.2002.38.4.668337
- "Sea Fresh Mouthwash", GNPD; MINTEL, 1 December 2006 (2006-12-01), XP002665823, [retrieved on 2006-12-01]
- ABE T ET AL: "Antimicrobial agent for elimination of microbes in waste water ports, comprises porous base material, as carrier, impregnated with plant extract e.g. aniseed oil, orange oil and/or cassia oil extract", WPI / THOMSON,, vol. 2002, no. 61, 28 May 2002 (2002-05-28), XP002638286,
- KALUS U ET AL: "Effect of Nigella sativa (black seed) on subjective feeling in patients with allergic diseases", PHYTOTHERAPY RESEARCH, JOHN WILEY & SONS LTD. CHICHESTER, GB, vol. 17, 1 January 2003 (2003-01-01), pages 1209-1214, XP002538025, ISSN: 0951-418X, DOI: 10.1002/PTR.1356

## Description

### TECHNICAL FIELD

The present invention relates generally to the use of a combination of a Perilla oil and sesame oil to inhibit activation of mast cells. In particular, the present invention relates to the use of a combination of a *Perilla* oil and sesame oil to prevent allergic rhinitis.

### BACKGROUND

Mast cells are granulocytes derived from myeloid stem cells. Mast cells are activated by allergens through cross-linking with IgE receptors on the surface of the cells, physical injury through damage-associated molecular pattern molecules, pathogen-related agents through pathogen-associated molecular pattern molecules, various compounds with their associated G-protein coupled receptors and complement proteins. Activation of mast cells causes a complex intracellular signalling pathway to occur, which results in degranulation of mast cells. This is where molecules stored in the granules of the mast cells are released outside of the cell. This includes, for example, serine proteases (e.g. tryptase), histamine, serotonin and proteoglycans (e.g. heparin). Mast cells play a role in the immune and neuroimmune systems, wound healing, angiogenesis and blood-brain barrier function. Mast cells have thus been implicated in numerous diseases.

Therefore, there is an ongoing need for compositions that inhibit mast cells, which may, for example, have prophylactic and/or therapeutic effects on disorders associated with mast cell activation and/or degranulation.

US 7,384,656 B2 describes a composition and related method that prevents, inhibits and/or mitigates an allergic response by down regulating the production of IgE, down regulating the binding of IgE antibodies to receptors on cells, and/or inhibiting allergy mediators, for example, histamine, prostoglandin D2, or luekotriene C4 release. The composition comprises at least one of the following ingredients: luteolin from Perilla leaf or seed, Cinnamon, Kiwi, Picao preto, Hesperidin, Acerola cherry, Guaco, Holy Basil, Kakadu, Solamum, Rosmarinic acid, Tinospora and Aframomum. JP-A-2000316473 describes a method for producing an edible oil comprising mixing one or more kinds of seeds selected from purified linseed seeds, purified Perilla ocimoides seeds, purified Perilla frutescens crispa seeds, purified Oenothera tetraptera seeds, an/or purified borage seeds with purified sesame seeds preferably in an amount of 10 to 80%, steaming the mixture by a conventional method, squeezing the steamed mixture, for example, by the use of an expeller, to obtain the squeezed raw oil, and then filtering the squeezed raw oil, when an edible oil characterized by a flavor is produced, or subjecting the squeezed raw oil to a degumming treatment, deacidification treatment, a decoloring treatment and a deodorizing treatment, when an edible salad oil, or the like, scarcely having a flavor is produced.

JP H04 290822 discloses a 1:1 combination of perilla oil and sesame oil (group E in example 3) which is shown to lead to an allergy preventive effect when administered orally in form of capsules.

### SUMMARY

The present invention aims, to provide a composition having a specific combination of active ingredients, which acts to inhibit mast cell activation.In certain embodiments, the present invention aims to provide a composition having a specific combination of active ingredients, which act synergistically, to inhibit mast cell activation and/or mast cell degranulation and/or release of histamine from mast cells.

According to a first aspect of the invention, there is provided a composition comprising a perilla oil and sesame oil, wherein the perilla oil and sesame oil are present in the composition in a weight ratio of about 1:1 for use in a method of preventing seasonal allergic rhinitis or perennial allergic rhinitis;
in the form of a spray.

The composition may inhibit degranulation of mast cells. The composition may inhibit release of histamine from mast cells.

The composition may reduce histamine release by at least about 20% or by at least about 40% or by at least about 50%. For example, the composition may reduce the concentration of histamine in the tissue of a subject by at least about 20% or by at least about 40% or by at least about 50%.

The present disclosure also provides a method for making a composition comprising a *Perilla* oil and sesame oil the method comprising combining the *Perilla* oil and the sesame oil.

Embodiments of the invention will be further described in the detailed description. Any embodiment described herein or any combination of embodiments described herein is applicable to any one or more aspects of the present invention unless clearly contradicted by context.

### BRIEF DESCRIPTION OF THE DRAWINGS

FIGURE 1 is a graph showing the percentages of histamine reduction from HMC-1 cell line when treated with perilla seed oil, sesame seed oil, cromolyn and the combination, as compared to the respective controls.
FIGURE 2 is a graph showing the percentages of histamine reduction from HMC-1 cell line when treated with the combination and Cromolyn, compared to the averaged histamine values of perilla and sesame oils.

### DETAILED DESCRIPTION

In the following disclosure, references to "a Perilla spp. extract" are to perilla oil, and references to a "another plant extract" are to sesame oil.

The present invention is based on, at least in part, the surprising finding that a combination of a *Perilla* spp. extract and another plant extract can inhibit mast cell activation. For example, embodiments of the present invention are based on the surprising finding that a *Perilla* ssp. extract and another plant extract may work synergistically to inhibit mast cell activation. In certain embodiments, the combination of a *Perilla* spp. extract and another plan extract can be used to treat disorders associated with mast cell activation and/or mast cell degranulation.

Hereinafter, the invention shall be described according to preferred embodiments of the present invention and by referring to the accompanying description and drawings. However, it is to be understood that limiting the description to the preferred embodiments of the invention is merely to facilitate discussion of the present invention and it is envisioned that those skilled in the art may devise various modifications without departing from the scope of the appended claim.

The terms generally used hereinbefore and hereinafter have for preference the meanings indicated below, unless indicated otherwise, whereby more specific meanings may be used independently of one another in preferred embodiments of the present inventions instead of the general definitions, these more specific significances describing especially preferred embodiments of the invention.

Where the term "at least one" or "one or more" occurs hereinbefore and hereinafter, this signifies in particular one to ten. for preference one to three, and in particular one or, further, two of the features enumerated, such as components, Where ranges are indicated, such as weight percentage ranges, these include the limit values indicated; thus, for example, "between X and Y" signifies "from and including X up to and including Y".

The term "product" or "composition" may mean in particular a pharmaceutical product or pharmaceutical composition in the sense of a formulation, whereby this term is not restricted to pharmaceutical products suitable for registration, or a medical product or fictitious pharmaceuticals, The "product" or "composition" may refer to a nutraceutical product in the sense of a formulation.

The term "anti-allergic" is to be understood to relate to an agent or measure that prevents, inhibits, or alleviates an allergic reaction.

The term "anti-infiammatory" is to be understood to relate to an agent or measure that prevents, inhibits, or alleviates an inflammatory reaction.

The term "therapeutic treatment" or "therapeutic method", also includes prophylaxis and the alleviation of symptoms in a subject, although not cosmetic treatments.

This relates to therapeutic treatment, and in particular prophylaxis of disorders relating to mast cells, for example disorders relating to mast cell activation and/or mast cell degranulation.

The components referred to hereinbefore and hereinafter are in particular selected from among those such as are listed in pharmacopoeia, e.g. in the US Pharmacopoeia National Formulary, the Pharmacopoea Europea, the Pharmacopoea Helvetica, the British Pharmacopoeia, the German Pharmacopoeia, the Japanese Pharmacopoeia, the Chinese Pharmacopoeia, or supplements, such as by way of decrees.

The present disclosure relates to a composition comprising a *Perilla* spp. extract and another plant extract. The composition may, for example, comprise one or more further plant extracts. For example, the composition may comprise a third or fourth or fifth or sixth plant extract. The composition may, for example, consist essentially of or consist of a *Perilla* spp. extract and another plant extract. The composition may, for example, consist essentially of or consist of a *Perilla* spp. extract and another plant extract and a third plant extract and optionally a fourth, fifth or sixth plant extract.

The term "extract" encompasses aqueous extract, non-aqueous extract, alcoholic extracts, evaporation, distillation (e.g. by water, steam, hydro diffusion, cohobation, rectification, fractional, etc), expression / cold pressing, supercritical CO₂ extraction, dilutions, plant concentrates, plant oils, plant macerations, plant exudates, plant resins, plant powders and plant granules and any combination of two or more thereof. However, the invention should not be construed as being limited to such embodiments. The *Perilla* spp. extract may, for example, be derived from *Perilla frutescens* (also known as perilla, Chinese basil and beefsteak), *Perilla citrodora, Perilla hirtella, Perilla setoyensis* or a combination of two or more thereof.

The other plant extract and the optional third, fourth, fifth or sixth plant extracts may, for example, each independently be an anti-inflammatory or an anti-allergic plant extract. The other plant extract and the optional third, fourth, fifth or sixth plant extracts may, for example, each independently be a mast cell stabilizing extract. The other plant extract and the optional third, fourth, fifth or sixth plant extracts may, for example, each independently be a medicinal or therapeutic or nutritional or health benefiting plant extract.

The optional third, fourth, fifth or sixth plant extracts may, for example, each be independently derived from *Nigella* spp. (e.g. *Nigella sativa* which is also known as black cumin, black caraway or fennel seed), *Urtica* spp. (e.g. nettle), *Astragalus* spp. (e.g. milk vetch), *Petasites* spp. (e.g. butterbur), *Citrus* spp. (e.g. lemon), *Uncaria* spp. (e.g. Cat's claw), *Lavandula* spp. (e.g. lavender), *Mentha* spp. (e.g. peppermint), *Eucalyptus* spp. (e.g. *Eucalyptus globulus* which is also known as Tasmanian blue gum, southern blue gum or blue gum), *Matricaria* spp. (e.g. German chamomile), *Rosmarinus* spp. (e.g. rosemary), *Curcuma* spp. (e.g. turmeric), *Allium* spp. (e.g. garlic), or a combination of any two or more thereof. The other (second) plant extract is derived from *Sesamum* spp.

The *Perilla* spp. extract, the other plant extract and the optional third, fourth, fifth and sixth plant extracts may, for example, each be independently obtained from the seed root, leaf, or whole of the plant as listed in the preceding description. In certain embodiments, the plant extracts are each independently (e.g. all) derived from the seed of the plant.

The *Perilla* spp. extract is perilla oil and the other plant extract is sesame oil. In certain embodiments, the *Perilla* spp. extract is perilla oil derived from the seed of the plant of *Perilla* spp., and the other plant extract is sesame oil. In certain embodiments, the *Perilla* spp. extract is *Perilla frutescens* seed oil and the other plant extract is *Sesamum indicum* seed oil.

The *Perilla* spp. extract is perilla oil and the other plant extract is sesame oil and the weight ratio of perilla oil to sesame oil is in the range of about 1:1.

The *Perilla* spp. extract and the other plant extract may, for example, be present in the composition in an amount by weight from about 5% to about 99.5% or from about 10% to about 95% or from about 20% to about 90% or from about 30% to about 80% or from about 40% to about 70% or from about 50% to about 60%. For example, the *Perilla* spp. extract and the other plant extract may, for example, be present in the composition in an amount by weight from about 20% to about 60% or from about 25% to about 55% or from about 30% to about 50% or from about 35% to about 45%.

The composition may be administered in the form of a composition comprising any suitable additional component.

The compositions take the form, of sprays.

Techniques and formulations generally may be found in Remington, The Science and Practice of Pharmacy, Mack Publishing Co., Easton, PA, latest edition.

The term "pharmaceutical composition" or "medicament" in the context of this invention means a composition comprising (a pharmaceutically effective amount of) a *Perilla* spp. extract and another plant extract, and additionally one or more pharmaceutically acceptable carriers and/or excipients. The pharmaceutical composition may further contain ingredients selected from, for example, diluents, adjuvants, excipients, vehicles, counterions, preserving agents, emulsifying agents, suspending agents, colouring agents, preserving agents, gelling agents, perfuming agents, antibacterial agents, antifungal agents, buffering agents.

The compositions may comprise no more than about 50 % w/w of pharmaceutically acceptable carrier and/or excipient, for example, no more than about 45 % w/w of pharmaceutically acceptable carrier and/or excipients, or no more than about 40 % of w/w pharmaceutically acceptable carrier and/or excipients, or no more than about 35 % w/w of pharmaceutically acceptable carrier and/or excipients. For example, the pharmaceutical composition may comprise at least about 1 % w/w, or at least about 10 % w/w, or at least about 15 % w/w, or at least about 20 % w/w, or at least about 25 % w/w, or at least about 30 % w/w of pharmaceutically acceptable carrier and/or excipients.

Liquid or semi-solid form preparations include solutions, suspensions, and emulsions, for example, water or water-propylene glycol solutions (for topical administration). Liquid or semi-solid preparations can also be formulated in solution in aqueous polyethylene glycol solution.

The active ingredient(s), i.e., plant extracts, may be mixed with one or more pharmaceutically acceptable carriers, such as water and/or any of the following: solvent such as propylene glycol, isopropyl myristate, alcohol; humectant such as glycerol; preservatives such as phenoxyethanol, caprylyl glycol, ethylhexylglycerin, benzalkonium chloride, potassium sorbate, disodium EDTA, benzoates and parabens; viscosity modifiers/thickeners such as gums, soybean oil, polyacrylates (e.g. sodium polyacrylate, Carbopol 980 polymer) and alginates; buffering agents such as monopotassium phosphate, di-potassium hydrogen phosphate and glycerin; gelling or emulsifying agents such as bentonite, xanthan gum, carrageenan, polysorbate 80, polysorbate 20, gelatin, cetearyl alcohol, lecithin, glycerol monostearate, antioxidants such as tocopherols and citric acid; colouring agents; pH regulators and surfactants.

The composition may further comprise other biologically active agents, for example, biologically active agents suitable for inhibiting mast cell activation or suitable for preventing or treating a disease or disorder associated with mast cell activation or mast cell degranulation. For example, the composition may further comprise other anti-allergy active agents.

The composition or pharmaceutical composition may further comprise a nutrient ingredient selected from the group consisting of vitamins and minerals, and combinations thereof. The vitamin may be any one or more of vitamin A, vitamin D, vitamin E, vitamin K, thiamine, riboflavin, pyridoxine, cyanocobalamin, carotenoids (including beta-carotene, zeaxanthin, lutein and lycopene), niacin, folic acid, pantothenic acid, biotin, vitamin C, choline, inositol, and salts and derivatives thereof. The mineral may be any one or more of calcium, phosphorous, magnesium, iron, zinc, manganese, copper, cobalt, boron, iodine, sodium, potassium, molybdenum, selenium, chromium, fluorine and chloride.

In certain embodiments, the plant extract components have a synergistic effect on inhibiting mast cell activation and/or mast cell degranulation and/or release of histamine from mast cells.

Mast cells are activated by allergens through cross-linking with IgE receptors on the surface of the cells, physical injury through damage-associated molecular pattern molecules, pathogen-related agents through pathogen associated molecular pattern molecules, various compounds with their associated G-protein coupled receptors and complement proteins. Activation of a mast cell causes a complex intracellular signalling pathway to occur, which results in degranulation of the mast cells. This is where molecules stored in the granules of the mast cell are released outside of the cell. This includes, for example, serine proteases (e.g. tryptase), histamine, serotonin and proteoglycans (e.g. heparin). Inhibiting activation of mast cells may, for example, include any one or more of these steps. For example, the compositions disclosed herein may be useful in inhibiting mast cell degranulation. For example, the compositions disclosed herein may be useful in inhibiting release of histamine from the mast cell.

Thus, in certain embodiments, the composition may be used to
reduce histamine release by at least about 20%. For example, the composition may reduce histamine release by at least about 30% or at least about 40% or at least about 50% or at least about 60% or at least about 70% or at least about 75% or at least about 80% or at least about 85% or at least about 90% or at least about 95%. For example, the composition may reduce histamine release by up to 100%, for example up to 99% or up to 98%. This may, for example, be determined by measuring histamine content in cell culture medium, for example as described in the examples below.

Also disclosed is use of a *Perilla* spp. extract and another plant extract in the manufacture of a medicament or pharmaceutical composition. The medicament or pharmaceutical composition is for preventing an allergic disorder.

Allergic disorders encompass disorders caused by hypersensitivity of the immune system. Generally, allergic disorders are caused by hypersensitivity to an allergen that causes little reaction in most subjects. Allergens that may be related to allergic disorders may be derived from, for example, foods, latex, medications, pollen and other animals (e.g. animal hair, insect stings). Allergic disorders encompass allergic disorders of the nasal cavity, including seasonal allergic rhinitis and perennial allergic rhinitis. Allergic disorders also include allergic conjunctivitis, allergic contact dermatitis, atopic dermatitis and allergic asthma. Anaphylaxis is a serious allergic reaction. In the present invention, the allergic disorder is an allergic disorder of the nasal cavity, namely seasonal allergic rhinitis and/or perennial allergic rhinitis.

The composition may reduce histamine concentration in the tissue of a subject by at least about 20%. For example, the composition may reduce histamine concentration in the tissue of a subject by at least about 30% or at least about 40% or at least about 50% or at least about 60% or at least about 70% or at least about 75% or at least about 80% or at least about 85% or at least about 90% or at least about 95%. For example, the composition may reduce histamine concentration in the tissue of a subject by up to 100%, for example up to 99% or up to 98%.

Histamine concentration in the tissue of a subject may be measured by any method known to a person skilled in the art. For example, histamine concentration may be determined by microdialysis sampling followed by quantification with commercially available ELISA kits or by HPLC. For example, histamine concentration at a localised site may be determined using tissue samples such as tissue homogenates. For example, histamine concentration may be determined using tissue from a site where an allergen has come into contact with mast cells or a sampling site (e.g. skin, mucosal lining (respiratory tract, gastrointestinal), blood, organs, bone marrow, etc).

The composition disclosed herein or the pharmaceutical composition disclosed herein can further comprise an anti-allergic drug or pharmaceutically active anti-allergic substance, for example an antihistamine or an additional mast cell stabilizer (e.g. cromoglicate, etc). The composition or the pharmaceutical composition can further comprise an antihistamine, including H₁-antihistamine (e.g. azelastine, olopatadine, triprolidine, bilastine, cetirizine, pyrilamine etc), H₂-antihistamine (e.g. cimetidine, famotidine, etc) and H₃-antihistamine (e.g. thioperamide, etc). The antihistamine opposes the activity of histamine receptors in a subject and further enhances the inhibitory effect of mast cell activation of the anti allergic oil and oily component (perilla oil and sesame oil) in the composition or the pharmaceutical composition. Azelastine can be used as the antihistamine in the composition, as it has a triple mode of action, i.e. antihistamine effect, mast cell stabilizing effect and anti-inflammatory effect. For example, the composition or the pharmaceutical composition may comprise from about 0.1 wt. % to about 50 wt. %, or from about 0.01 wt. % to about 10 wt. %, or from about 10 wt. % to about 40 wt. %, or from about 20 wt. % to about 30 wt. % of antihistamine by weight of the composition described herein.

In certain embodiments, the subject is a human. In other embodiments, the subject is a mammal other than a human, such as non-human primates (e.g. apes, monkeys and lemurs), companion animals such as cats or dogs, working and sporting animals such as dogs, horses and ponies, farm animals such as pigs, sheep, goats, deer, oxen and cattle, and laboratory animals such as rodents (e.g. rabbits, rats, mice, hamsters, gerbils or guinea pigs).

In certain embodiments, the composition is administered daily to the subject (e.g. orally or topically administered daily to the subject). The amount of composition administered may be varied depending upon the requirements of the subject. For therapeutic applications, the amount of composition administered may be varied depending upon the desired results, the requirements of the subject and the severity of the condition being treated. Determination of the proper amount/dosage for a particular situation is within the skill of the art. For example, for therapeutic applications a physician or veterinarian having ordinary skill in the art can readily determine and prescribe the effective amount of the pharmaceutical composition required. The total daily amount/dosage may be divided and administered in portions during the day if desired.

In general, a suitable daily dose of active agents in the composition
will be that amount which is the lowest dose effective to produce the desired effect, for example, a therapeutic effect, and/or to inhibit mast cell activation and/or to inhibit mast cell degranulation and/or to inhibit release of histamine from mast cells. It is contemplated that a wide range of doses may be used, due to the non-toxic nature of the composition. A person of ordinary skill in the art will understand that a suitable dose or dosage will typically vary from subject to subject, and will be dependent on factors such as the severity of health conditions of the subject at the outset of administration of the composition. For example, the dose of active agents (i.e. plant extracts) in the composition may be up to 15 g per day, for example, up to about 10 g per day, or up to about 5 g per day. In certain embodiments, the doses of active agents in the composition is in the range of 100 mg to about 3 g per day, which may be administered as two or three or more sub-doses administered separately at appropriate intervals throughout the day, optionally in unit dosage forms. In certain embodiments, the dose of active agents in the composition may be from about 10 mg to about 3 g of each extract component per day, for example, from about 500 mg to about 3 g of each component per day, or from about 750 mg to about 2.5 g of each component per day, or from about 1000 mg to about 2000 mg of each component per day. In certain embodiments, the composition may be administered two or three times a day, optionally before, with, or after a meal. In certain embodiments, each dose of active agents is no more than about 5 g, for example, no more than about 3 g, for example, no more than about 2.5 g. Each dose of the plant extracts in the composition may be combined with other conventional agents for inhibiting mast cell activation.

The compositions and pharmaceutical compositions described herein may be prepared by combining a *Perilla* spp. extract and a second plant extract and optionally any one or more of the other ingredients described herein. such as one or more further plant extracts, biologically active agents and pharmaceutical excipients and/or carriers and/or diluents. The components are combined in suitable amounts to obtain a composition having the desired quantity of each component Each component may be combined with one or more other components in any order and combination suitable to obtain the desired product. Such methods are well known in the art, for example, methods known in the pharmaceutical industry.

The preparations can be manufactured according to conventional and known methods, which Include in particular the mixing of the components, if required step by step and/or under movement and/or heating of the fluid, for example by stirring.

The invention will now be described in detail by way of reference only to the following non-limiting examples.

### EXAMPLES Example 1: Mast Cell Test

Mast cell lines, i.e. HMC1 cells (provided by Dr Joseph H. Buttetfield, Mayo Clinic, Rochester, MN, USA), were cultured in Isocove's Modified Dulbecco's Medium (IMDM) containing 10 % Fetal Bovine Seum (FBS), 2 mM L-glutamine, 50 *µ*M 2-mercaptoethanol, 100 U/mL penicillin and 100 *µ*g/mL streptomycin. Cells were maintained at 37°C in 5% CO₂ in a humidified atmosphere.

Percentage of living cells was determined by trypan blue staining. A 1:10 dilution with 50 *µ*l cell suspension and 450 *µ*l trypan blue was prepared and the number of living and dead cells was counted in a hemocytometer. 10⁶ living cells were transferred into safe-lock Eppendorf tubes, Cell suspensions were centrifuged (200 g, 5 min) and supernatant was discarded.

The effect of perilla oil, sesame oil and a combination of perilla oil and sesame oil (1:1 ratio) in the vehicle Kolliphor ELP (polyoxyl-35 hydrogenated castor oil) was determined. The positive control Crortiolyti in phosphate buffer saline (PBS), the vehicles PBS and ELP were also tested as controls.

After adding 100 *µ*l IgE (100 ng/ml in PBS or ELP) to the cell pellets, cells were incubated for 12 hours over night, at 37°C. Cells were then incubated for 2 hours, at 37°C with two different concentrations of compounds, i.e, 250*µ*g/ml and 500 *µ*g/ml. After centrifugation (200 g, 5 min) supernatant was discarded and cell pellet was again incubated for 1 hour, at 37°C with 100 *µ*l anti-IgE (1 *µ*g/ml in PBS or ELP), For the final incubation time of 6 hours, at 37°C, 300 *µ*l HMC1 media was added. Cells were again centrifuged (200 g, 5 min) and supernatant, was collected and stored as 300 *µ*l aliquots at -20°C for ELISA measurements. Cell pellets were resuspended in 100 *µ*l HMC1 media for determining cell viability.

Viability of the cells (percentage of living cells) was determined by trypan blue staining (1:10 dilution with 50 *µ*l cell suspension and 450 *µ*l trypan blue was prepared and the number of living and dead cells was counted in a hemocytometer).

The concentration of histamine in the collected supernatant was determined by ELISA, using a kit obtained from IBL International according to the manufacturer's guidelines, Each sample was measured twice, Due to expected measuring limits, vehicle (PBS, ELP) treated samples were diluted 1:2.

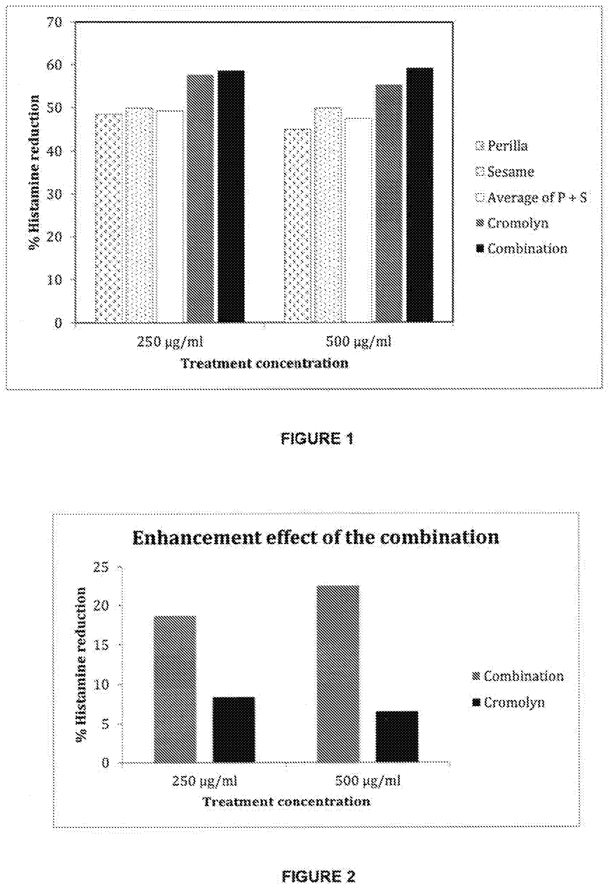

Statistical analysis was performed with GraphPad Prism (Version 5,01) software (GraphPad Software Inc., San Diego, CA, USA) using the unpaired two-tailed Students t test A *P* value of less than 0.05 was considered statistically significant The results are shown in Figures 1 and 2, as well as Tables 1 and 2 below.

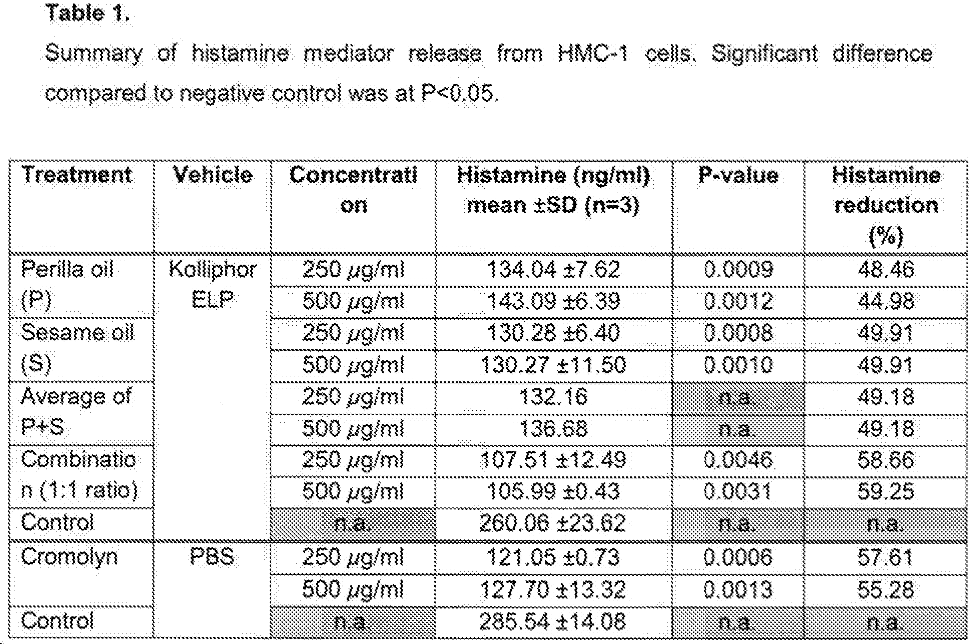

**Table 2.**

| Histamine reduction compared to averaged histamine mediator release of perilla oil and sesame oil from HMC-1 cells, showing enhancement effect of the combination. Significant difference compared to averaged values of perilla oil and sesame oil was at P<0.05. | | | |
|---|---|---|---|
| **Treatment** | **Concentration** | **Histamine reduction (%)** | **P-value** |
| Combination | 250 *µ*g/ml | 18.65 | 0.0185 |
| | 500 *µ*g/ml | 22.46 | 0.0092 |
| | 250 *µ*g/ml | 8.41 | 0.0668 |
| | 500 *µ*g/ml | 6.75 | 0.0728 |

Comparing the average histamine release of perilla oil and sesame oil to the combination, the combination showed significantly reduced histamine release and therefore mast cell stabilization at 250 and 500 *µ*g/ml (p=0.0185 and p=0.0092) respectively.

When HMC-1 cells were treated individually with perilla oil or sesame oil, the percentage of histamine reduction achieved was lower than that of Cromolyn. When treated with the combination, the percentage of histamine reduction was higher than treatment with Cromolyn (Figure 1). Treatment with the combination Increased the percentage of histamine reduction by 18.7 - 22.5% compared to treatment with individual compounds, while treatment with Cromolyn only differed by 6.8 - 8.4% of histamine reduction compared to treatment with individual compounds.

There is an approximately 2-fold and 3~fold difference between the combination and Cromolyn at concentrations of 250 *µ*g/ml and 500 *µ*g/ml respectively. It can be concluded that the combination had enhanced mast cells stabilizing effect, which increased the percentage of histamine reduction compared to treatment with Individual compounds, and reached a final effect that was better than Cromolyn.

### Example 2: Anti-altergic Formulation (not covered by the claims)

Tables 3 - 5 show anti-allergic pill formulations in a hard or soft shell capsule.

**Table 3.**

| **Ingredient** | **Percentage (%) by weight** |
|---|---|
| Perilla oil | 49.75 |
| Sesame oil | 49.75 |
| Tocopherol | 0.5 |

**Table 4.**

| **Ingredient** | **Percentage (%) by weight** |
|---|---|
| Perilla oil | 20.5 |
| Urtica extract | 13.8 |
| Astragalus extract | 122 |
| Nigella oil | 18.5 |
| Soybean oil | 30 |
| Lecithin | 5 |

**Table 5.**

| **Ingredient** | **Percentage (%) by weight** |
|---|---|
| Perilla extract | 35.3 |
| Urtica extract | 25.4 |
| Astragalus extract | 24.8 |
| Maltodextrine | 14.5 |

**Table 6.**

| **Ingredient** | **Percentage (%) by weight** |
|---|---|
| Perilla oil | 49.0 |
| Sesame oil | 49.0 |
| Cetirizine hydrochloride | 1.0 |
| Citric acid | 1.0 |

Tables 7 and 8 show an anti-allergic topical gel formulation.

**Table 7.**

| **Ingredient** | **Percentage (%) by weight** |
|---|---|
| Perilla oil | 15 |
| Sesame oil | 5 |
| Disodium EDTA | 0.1 |
| Menthol | 0.5 |
| Propylene glycol | 12 |
| Carbopol 980 polymer | 0.85 |
| Polysorbate 80 | 1.5 |
| myristate | 2.0 |
| Citric acid | 0.1 |
| Water | 62.95 |

**Table 8.**

| **Ingredient** | **Percentage (%) by weight** |
|---|---|
| Perilla oil | 15 |
| Sesame oil | 5 |
| Disodium EDTA | 0.1 |
| Menthol | 0.5 |
| Propylene glycol | 12 |
| Carbopol 980 polymer | 0.85 |
| Polysorbate 80 | 1.5 |
| myristate | 2.0 |
| Citric acid | 0.1 |
| Azelastine hydrochloride | 2.0 |
| Water | 60.95 |

Tables 9 and 10 show an anti-allergic lotion formulation.

**Table 9.**

| **Ingredient** | **Percentage (%) by weight** |
|---|---|
| Perilla oil | 10 |
| Sesame oil | 20 |
| Spearmint oil | 0.1 |
| Water | 62.5 |
| Glycerin | 5 |
| Xanthan gum | 0.5 |
| Carrageenan | 0.3 |
| Tocopherol | 0.5 |
| Phenoxyethanol | 1 |
| Ethylhexylglycarin | 0.1 |

**Table 10.**

| **Ingredient** | **Percentage (%) by weight** |
|---|---|
| Perilla oil | 5 |
| Sesame oil | 35 |
| Purified water | 40.9 |
| Glycerin | 10 |
| Citric acid | 2 |
| Cetearyl alcohol | 1 |
| Polysorbate 20 | 1 |
| Aloe vera | 5 |
| Vanilla oil | 0.1 |
| Potassium sorbate | 1 |

Table 11 shows an anti-allergic ointment formulation.

**Table 11.**

| **Ingredient** | **Percentage (%) by weight** |
|---|---|
| Perilla oil | 20 |
| Sesame oil | 20 |
| Menthol | 5 |
| Ointment base | 55 |

Table 12 shows an anti-allergic gel patch formulation.

**Table 12.**

| **Ingredient** | **Percentage (%) by weight** |
|---|---|
| Perilla oil | 35 |
| Sesame oil | 5 |
| Water | 20 |
| Glycerin | 20 |
| Propylene glycol | 10 |
| Hyaluronic acid | 7 |
| Potassium sorbate | 1 |
| Sodium polyacrylate | 2 |

Tables 13, 14 and 15 show anti-allergic pill formulations in a tablet.

**Table 13.**

| **Ingredient** | **Percentage (%) by weight** |
|---|---|
| Perils extract | 38.5 |
| Urtica extract | 30.8 |
| Astragalus extract | 15.4 |
| Croscarmellose sodium | 7.7 |
| Cellulose | 6.8 |
| Silicon dioxide | 0.5 |
| Magnesium stearate | 0.3 |

**Table 14.**

| **Ingredient** | **Percentage (%) by weight** |
|---|---|
| Perilla extract | 25.3 |
| Urtica extract | 25.3 |
| Astragalus extract | 25.3 |
| Croscarmellose sodium | 1.5 |
| Cellulose microcrystalline | 20.6 |
| Silicon dioxide | 0.5 |
| Magnesium stearate | 1.5 |

**Table 15.**

| **Ingredient** | **Percentage (%) by weight** |
|---|---|
| Perilla extract | 25.3 |
| Urtica extract | 25.3 |
| Astragalus extract | 25.3 |
| Croscarmellose sodium | 1.5 |
| Cellulose microcrystalline | 20.1 |
| Silicon dioxide | 0.5 |
| Magnesium stearate | 1.5 |
| Bilastine | 0.5 |

## Claims

1. A composition in the form of a spray, the composition comprising a perilla oil and sesame oil, wherein the perilla oil and sesame oil are present in the composition in a weight ratio of about 1:1, for use in a method of preventing seasonal allergic rhinitis or perennial allergic rhinitis.

2. The composition for use according to claim 1, wherein the composition is a pharmaceutical composition, optionally further comprising a pharmaceutically acceptable carrier, excipient, diluent, and/or additive.

## Patentansprüche

1. Zusammensetzung in Form eines Sprays, wobei die Zusammensetzung ein Perillaöl und Sesamöl umfasst, wobei das Perillaöl und das Sesamöl in der Zusammensetzung in einem Gewichtsverhältnis von etwa 1:1 vorliegen, zur Verwendung in einem Verfahren zum Vorbeugen saisonaler allergischer Rhinitis oder ganzjähriger allergischer Rhinitis.

2. Zusammensetzung zur Verwendung nach Anspruch 1, wobei die Zusammensetzung eine pharmazeutische Zusammensetzung ist, die optional ferner ein(en) pharmazeutisch verträgliche(n/s) Träger, Hilfsstoff, Verdünnungsmittel und/oder Zusatzstoff umfasst.

## Revendications

1. Composition sous la forme d'une pulvérisation, la composition comprenant une huile de périlla et une huile de sésame, dans laquelle l'huile de périlla et l'huile de sésame sont présentes dans la composition dans un rapport en poids d'environ 1:1, pour une utilisation dans une méthode de prévention de la rhinite allergique saisonnière ou de la rhinite allergique apériodique.

2. Composition pour une utilisation selon la revendication 1, dans laquelle la composition est une composition pharmaceutique, comprenant en outre facultativement un vecteur, un excipient, un diluant et/ou un additif pharmaceutiquement acceptables.
